# EUROPEAN PATENT APPLICATION

(11) **EP 4 644 357 A1**
(43) Date of publication of application: **05.11.2025**
(21) Application number: 23912015.7
(22) Date of filing: 25.12.2023
(51) Int. Cl.: C07C 59/70, A61K 31/192, A61P 3/04, A61P 3/06, A61P 5/14, C07C 59/56

(54) **COMPOUND USEFUL AS THYROID HORMONE RECEPTOR B-SELECTIVE THYROID HORMONE ANALOG**

(30) Priority: 26.12.2022 JP 2022208167
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: YOSHIMURA, Takashi, Nagoya-shi, Aichi 464-8601 (JP); CRUDDEN, Cathleen, Nagoya-shi, Aichi 464-8601 (JP); NAMBO, Masakazu, Nagoya-shi, Aichi 464-8601 (JP); NISHIWAKI OHKAWA, Taeko, Nagoya-shi, Aichi 464-8601 (JP); SATO, Ayato, Nagoya-shi, Aichi 464-8601 (JP); ARIKI, Zachary T., Nagoya-shi, Aichi 464-8601 (JP); YAR, Muhammad, Nagoya-shi, Aichi 464-8601 (JP); YIM, Jacky C.-H., Nagoya-shi, Aichi 464-8601 (JP)
(74) Representative: Hoffmann Eitle
(86) International application number: PCT/JP2023/046295
(87) International publication number: WO 2024/143235

(57) **Abstract**

Provided is a compound useful as a thyroid hormone receptor β-selective thyroid hormone analog. A compound represented by Formula (1), a salt thereof, or a solvate thereof is provided.

## Description

### Technical Field

The present invention relates to compounds that are useful as thyroid hormone receptor β-selective thyroid hormone analogs.

### Background Art

The worldwide prevalence of obesity doubled from 1980 to 2015, with nearly 10% of the global population now classified as obese or overweight. Obesity is known to adversely affect the body's physiological functions and increases the risk of developing multiple diseases, such as diabetes, cardiovascular disease, and cancer.

In recent years, thyroid hormones (TH) have gained attention as a new treatment option for obesity. The action of thyroid hormones is mediated by their receptors (THRs), which belong to the superfamily of nuclear receptors. Thyroxine (T₄), a prohormone produced in the thyroid gland, is converted to biologically active triiodothyronine (T₃) by iodothyronine deiodinases 1 and 2 (DIO1 and DIO2). THR forms a complex with the retinoid X receptor (RXR) and corepressors, such as NcoR and SMRT, on the thyroid hormone-responsive elements (TRE) of target genes. The binding of T₃ to THR dissociates corepressors and allows coactivators, such as peroxisome proliferator-activated receptors (PPAR) α and γ, to work, leading to the transcriptional activation of target genes.

There are two subtypes of THRs: α and β. The subtype α (THRα) is highly expressed in the brain, heart, and muscles, while the subtype β (THRβ) is predominantly expressed in the liver. T₃ binding to THRα leads to side effects, such as cardiac arrhythmia and decreased muscle and bone volume, while T₃ binding to THRβ activates lipid metabolism in the liver and adipose tissue, resulting in decreased blood lipid levels. While the biological functions induced by T₃ binding to THRβ are beneficial for combating obesity, T₃ shows little selectivity between receptors, making it difficult to avoid serious side effects resulting from its binding to THRα.

To overcome this problem, efforts have been made to develop THRβ-selective TH analogs by derivatizing T₃. GC-1 is a representative THRβ-selective TH analog, showing 10-fold lower affinity for THRα than T₃ without significantly compromising its affinity for THRβ (NPL 1). Although the effects of GC-1 on lipid metabolism have been demonstrated in numerous in vitro and animal models, clinical trials were ended after phase 1.

### Citation List

### Non-patent Literature

NPL 1: Joharapurkar AA, Dhote VV, Jain MR. Selective thyromimetics using receptor and tissue selectivity approaches: prospects for dyslipidemia. J Med Chem. 55, 5649-5675 (2012).

### Summary of Invention

### Technical Problem

An object of the present invention is to provide a compound that is useful as a thyroid hormone receptor β-selective thyroid hormone analog.

### Solution to Problem

The present inventors began their research by designing novel THRβ-selective TH analogs based on the key structural features of GC-1 and GC-24. Their hydrophobic isopropyl and benzyl groups at the 3' position of the phenol moiety were considered important for high THRβ selectivity. In particular, X-ray crystallography analysis of THRβ and TH analogs suggests that these substituents are located in the hydrophobic region of the hormone-binding pocket and are thought to improve compatibility and affinity for THRβ. The 3,5-dimethyl substituents are crucial structural elements that fix the two aromatic rings in a perpendicular arrangement, positioning the molecule in the active conformation seen in effective TH analogs. Therefore, the inventors focused on introducing an aromatic ring to enhance hydrophobic interactions with THRβ. Furthermore, they focused on using bulky substituents, such as trifluoromethyl groups, to provide a more rigid conformation.

In light of the problem above, the present inventors conducted further intensive research and found that the compound represented by Formula (1) can solve the problem. As a result of continued research based on this finding, they completed the present invention. Specifically, the present invention encompasses the following aspects.

### Item 1.

A compound represented by Formula (1), a salt thereof, or a solvate thereof: 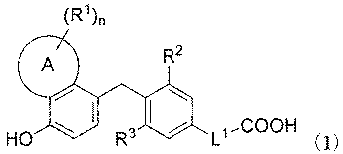 wherein
A represents an aromatic ring,
R¹ is the same or different, and represents a hydrocarbon group,
n represents an integer of 0 to 6,
R² and R³ are the same or different, and represent a trihalomethyl group or an alkyl group, and
L¹ represents a linker.

### Item 2.

The compound, salt thereof, or solvate thereof according to Item 1, wherein R² and R³ are the same or different, and represent a trihalomethyl group or a C₁-C₆ alkyl group.

### Item 3.

The compound, salt thereof, or solvate thereof according to Item 1, wherein the compound is represented by Formula (1A): 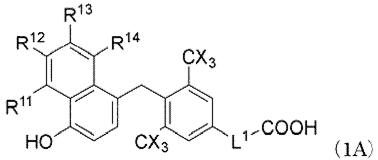 wherein
R¹¹, R ¹², R¹³, and R¹⁴ are the same or different, and represent a hydrogen atom or a hydrocarbon group,
X is the same or different, and represents a halogen atom, and
L¹ represents a linker.

### Item 4.

The compound, salt thereof, or solvate thereof according to Item 3, wherein the number of atoms that constitute the main chain of the linker is 1 to 4.

### Item 5.

The compound, salt thereof, or solvate thereof according to Item 3, wherein the compound is represented by Formula (1AA): 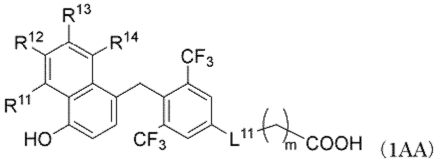 wherein
R¹¹, R¹², R¹³, and R¹⁴ are the same or different, and represent a hydrogen atom or a hydrocarbon group,
L¹¹ represents a single bond or -O-, and
m represents an integer of 1 to 3.

### Item 6.

A thyroid hormone analog consisting of the compound, salt thereof, or solvate thereof of any one of Items 1 to 5.

### Item 7.

A lipid metabolism improver comprising the compound, salt thereof, or solvate thereof of any one of Items 1 to 5.

### Item 8.

The lipid metabolism improver according to Item 8, for use in the improvement of at least one condition selected from the group consisting of obesity, dyslipidemia, and metabolic syndrome.

### Advantageous Effects of Invention

The present invention provides a compound useful as a thyroid hormone receptor β-selective thyroid hormone analog.

### Brief Description of Drawings

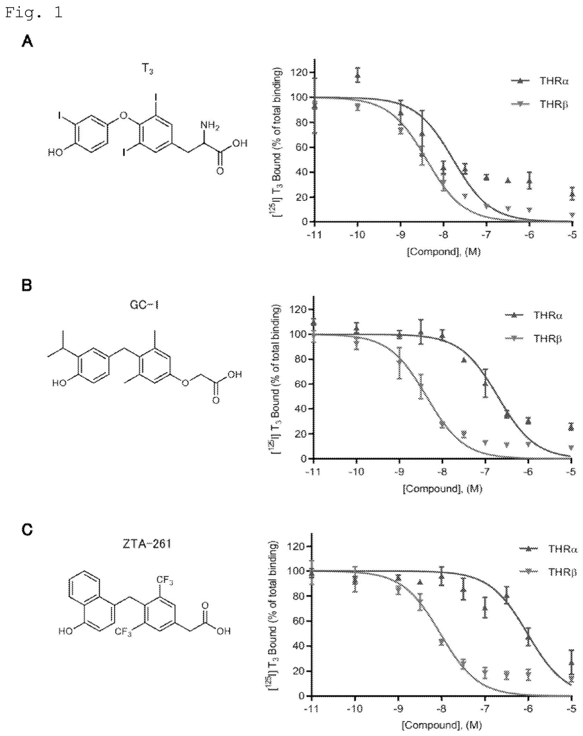

Fig. 1 shows the results of the radioligand-displacement assay in Test Example 2. Figs. 1A to 1C show the structural formulas of the test compounds on the left side. In Figs. 1A to 1C, the vertical axis shows the percentage of radioactively labeled T₃ bound to THRα or THRβ, and the horizontal axis shows the concentration of non-radioactive test compounds. Data are shown as the mean ± SD (n=3). The data were fitted using a one-site competition model.

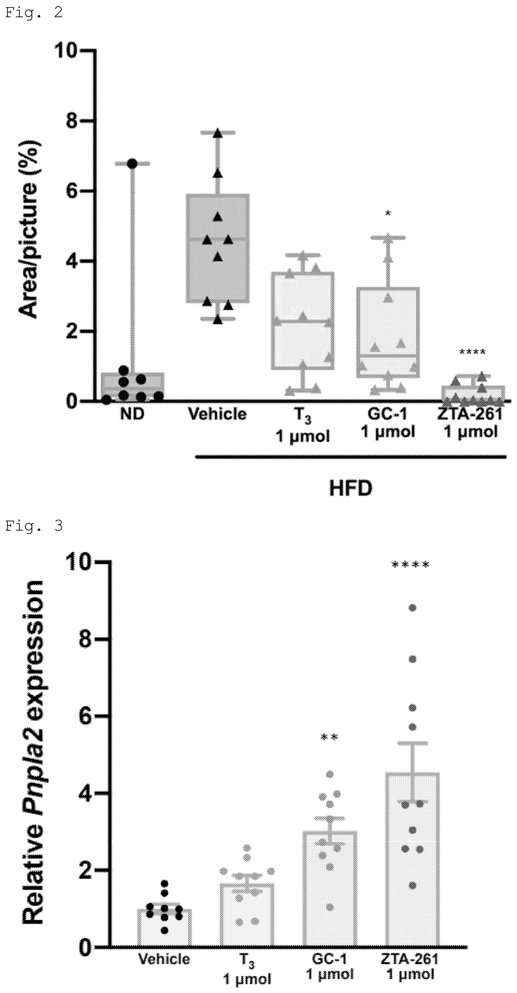

Fig. 2 shows the results of Oil Red O staining of liver sections from Test Example 4. The vertical axis shows the percentage of Oil Red O-positive areas. On the horizontal axis, "ND" indicates a normal diet, "HFD" indicates a high-fat diet, "Vehicle" indicates the group not treated with the test compounds, and other labels indicate groups treated with various test compounds. The average of three sections per mouse was plotted. Data are shown as box-and-whisker plots, with the median, 25% and 75% (n = 8 to 10, *P <0.05, ****P <0.0001, Kruskal-Wallis test with Dunn's multiple comparisons test).
Fig. 3 shows the results of gene expression measurement (Pnpla2 gene) in Test Example 5. The vertical axis shows the relative value of Pnpla2 gene expression to GAPDH gene expression. On the horizontal axis, "Vehicle" indicates the group not treated with the test compounds, while other labels represent the groups treated with various test compounds. Data are shown as the mean ± SEM (n = 7 to 10, ***P* <0.01, *****P* <0.0001 vs vehicle by one-way ANOVA with Dunnett's post-hoc analysis).

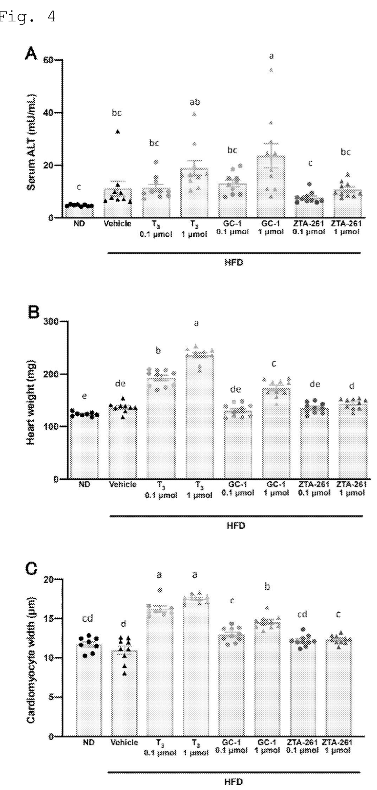

Fig. 4A shows the measurement results of serum ALT levels (vertical axis) in Test Example 6. Fig. 4B shows the measurement results of heart weight (vertical axis) in Test Example 7. Fig. 4C shows the measurement results of transnuclear width of left ventricular cardiomyocytes (vertical axis) in Test Example 8. On the horizontal axis, "ND" indicates a normal diet, "HFD" indicates a high-fat diet, "Vehicle" indicates the group not treated with the test compounds, and other labels represent the groups treated with various test compounds. Data are shown as the mean ± SEM (n = 8 to 10). Significant differences between groups are indicated with different letters (P < 0.05, one-way ANOVA with Tukey's post-hoc test).

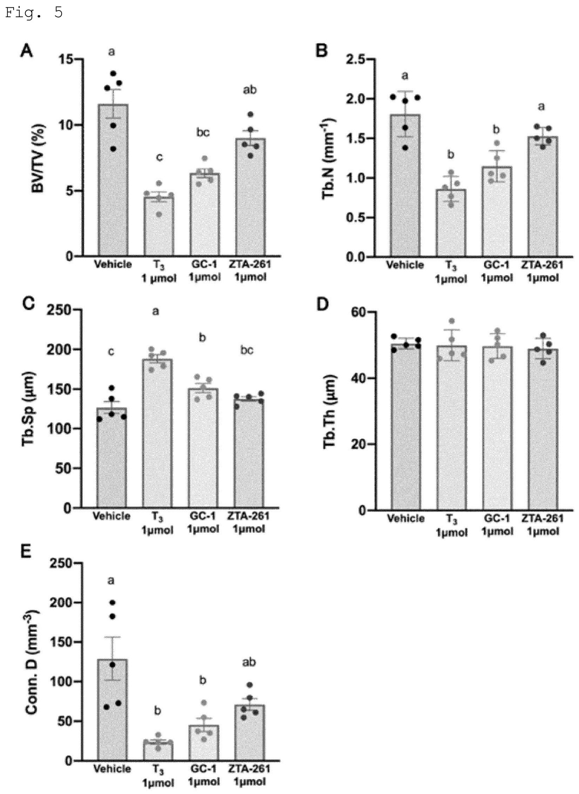

Fig. 5 shows the measurement results of bone structure parameters in Test Example 9. Fig. 5A shows the measurement results for bone volume (BV/TV). Fig. 5B shows the measurement results for trabecular number (Tb.N). Fig. 5C shows the measurement results for trabecular separation (Tb.Sp). Fig. 5D shows the measurement results for trabecular thickness (Tb.Th). Fig. 5E shows the measurement results for connectivity density (Conn.D). On the horizontal axis, "Vehicle" indicates the group not treated with the test compounds, while other labels indicate groups treated with various test compounds. Data are shown as the mean ± SEM (n = 5). Significant differences between groups are indicated with different letters (P < 0.05, one-way ANOVA with Tukey's post-hoc test).

### Description of Embodiments

In the present specification, the terms "comprising" and "containing" include the concepts of comprising, containing, consisting essentially of, and consisting of.

### 1. Compound

In one aspect, the present invention relates to a compound represented by Formula (1): 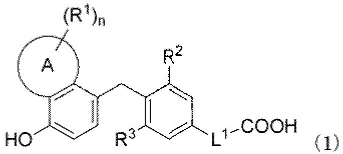 (which may also be referred to as "the compound of the present invention" in the present specification), a salt thereof, or a solvate thereof (which may be collectively referred to as "the active ingredient of the present invention" in the present specification). The following describes the compound, salt thereof, and solvate thereof.

A represents an aromatic ring.

The aromatic ring is not particularly limited and includes both an aromatic ring consisting only of hydrocarbons and an aromatic ring containing heteroatoms other than carbon and hydrogen (heterocyclic ring). The heteroatoms included in the heterocyclic ring are not particularly limited, and examples include sulfur atoms, nitrogen atoms, oxygen atoms, etc. The aromatic ring includes both a monocyclic ring (e.g., 4- to 8-membered rings) and a fused ring (e.g., bicyclic or tricyclic rings). Examples of aromatic rings include monocyclic rings, such as benzene, thiophene, thiazole, furan, pyrrole, imidazole, pyrazole, oxazole, isoxazole, triazole, tetrazole, pyridine, pyrazine, pyrimidine, pyridazine, and triazine; bicyclic rings, such as naphthalene, benzothiophene, benzofuran, indole, benzimidazole, indazole, benzoxazole, benzothiazole, isobenzofuran, isoindole, purine, quinoline, isoquinoline, quinoxaline, quinazoline, cinnoline, and phthalazine; and tricyclic rings, such as anthracene and phenanthrene.

From the viewpoint of THRβ selectivity, the aromatic ring preferably has a highly hydrophobic structure. From this viewpoint, the aromatic ring is preferably an aromatic ring consisting of carbon atoms and hydrogen atoms, more preferably benzene, naphthalene, anthracene, phenanthrene, etc., and particularly preferably benzene. For example, when the aromatic ring represented by A is benzene, partial structure (1a) in Formula (1): 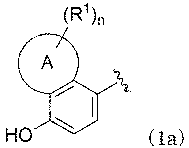 becomes partial structure (1a').

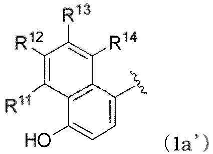

The definitions of R¹¹, R¹², R¹³, and R¹⁴ will be described below.

R¹ is the same or different, and represents a hydrocarbon group.

The hydrocarbon group represented by R¹ is not particularly limited, and examples include an alkyl group, an aryl group, and a group formed of these groups in any combination (e.g., an aralkyl group, an alkyl aryl group, and an alkyl aralkyl group). The hydrocarbon group is preferably an alkyl group or an aralkyl group.

The alkyl group represented by R¹ includes a linear alkyl group, a branched alkyl group, and a cyclic alkyl group. The number of carbon atoms in the alkyl group (in the case of a linear or branched alkyl group) is not particularly limited, and can be, for example, 1 to 8. The number of carbon atoms is preferably 1 to 5, more preferably 1 to 3, still more preferably 1 to 2, and even more preferably 1. The number of carbon atoms in the alkyl group (in the case of a cyclic alkyl group) is not particularly limited, and can be, for example, 3 to 7, and preferably 4 to 6. Specific examples of such alkyl groups include a methyl group, an ethyl group, an n-propyl group, an isopropyl group, an n-butyl group, an isobutyl group, a tert-butyl group, a sec-butyl group, an n-pentyl group, a neopentyl group, an n-hexyl group, a 3-methylpentyl group, an n-heptyl group, and an n-octyl group.

The aryl group represented by R¹ is not particularly limited, and an aryl group with 6 to 12 carbon atoms is preferred, an aryl group with 6 to 10 carbon atoms is more preferred, and an aryl group with 6 to 8 carbon atoms is even more preferred. The aryl group may be monocyclic or polycyclic (e.g., bicyclic or tricyclic), and is preferably monocyclic. Specific examples of such aryl groups include a phenyl group, a naphthyl group, a biphenyl group, a pentalenyl group, an indenyl group, an anthranyl group, a tetracenyl group, a pentacenyl group, a pyrenyl group, a perylenyl group, a fluorenyl group, and a phenanthryl group, with a phenyl group being preferred.

The aralkyl group represented by R¹ is not particularly limited, and examples include an aralkyl group in which one or more hydrogen atoms (e.g., preferably 1 to 3, more preferably 1 hydrogen atom) of a linear or branched C₁-C₆ (preferably C1-C₃, more preferably C₁-C₂) alkyl group are replaced with the aryl group described above. Specific examples of such aralkyl groups include a benzyl group and a phenethyl group.

The alkyl aryl group represented by R¹ is not particularly limited, and examples include an alkyl aryl group in which one or more hydrogen atoms (e.g., 1 to 3, or preferably 1 hydrogen atom) of the aryl group described above are replaced with a linear or branched C₁-C₆ (preferably C₁-C₂) alkyl group. Specific examples of such alkyl aryl groups include a tolyl group and a xylyl group.

The alkyl aralkyl group represented by R¹ is not particularly limited, and examples include an alkyl aralkyl group in which one or more hydrogen atoms (e.g., 1 to 3, or preferably 1 hydrogen atom) on the aromatic ring of the aralkyl group described above are replaced with a linear or branched C₁-C₆ (preferably C₁-C₂) alkyl group.

n represents an integer of 0 to 6.

From the viewpoint of THRβ selectivity, n is preferably an integer of 0 to 3, more preferably 0 to 1, and even more preferably 0.

In partial structure (1a'), R¹¹, R¹², R¹³, and R¹⁴ are the same or different, and represent a hydrogen atom or a hydrocarbon group. The hydrocarbon group is the same as the hydrocarbon group represented by R¹ described above. From the viewpoint of THRβ selectivity, the number of hydrocarbon groups in R¹¹, R¹², R¹³, and R¹⁴ is preferably 0 to 3, more preferably 0 to 1, and even more preferably 0.

R² and R³ are the same or different, and represent a trihalomethyl group or an alkyl group.

Examples of the trihalomethyl group represented by R² or R³ include a trifluoromethyl group, a trichloromethyl group, and a tribromomethyl group.

The alkyl group represented by R² or R³ includes a linear alkyl group, a branched alkyl group, and a cyclic alkyl group. The number of carbon atoms in the alkyl group (in the case of a linear or branched alkyl group) is not particularly limited, and can be, for example, 1 to 8. The number of carbon atoms is preferably 1 to 6, more preferably 1 to 4. The number of carbon atoms in the alkyl group (in the case of a cyclic alkyl group) is not particularly limited, and can be, for example, 3 to 7, and preferably 4 to 6. Specific examples of such alkyl groups include a methyl group, an ethyl group, an n-propyl group, an isopropyl group, an n-butyl group, an isobutyl group, a tert-butyl group, a sec-butyl group, an n-pentyl group, a neopentyl group, an n-hexyl group, a 3-methylpentyl group, an n-heptyl group, and an n-octyl group.

From the viewpoint of THRβ selectivity, R² and R³ preferably have a bulky structure. From this viewpoint, R² and R³ are preferably a trihalomethyl group or a branched alkyl group, more preferably a trihalomethyl group, an isopropyl group, or a tert-butyl group, even more preferably a trihalomethyl group or an isopropyl group, still more preferably a trihalomethyl group, and yet more preferably a trifluoromethyl group.

L¹ represents a linker.

The linker includes a chain structure. The chain structure is not particularly limited, and examples include chain structures with the number of atoms that constitute the main chain being, for example, 1 to 6; and from the viewpoint of THRβ selectivity, chain structures with the number of atoms that constitute the main chain being preferably 1 to 4, more preferably 1 to 3, even more preferably 1 to 2, and yet more preferably 1. Examples of atoms that constitute the main chain include a carbon atom, an oxygen atom, a sulfur atom, and a nitrogen atom. A part of the chain structure or the entire chain structure may be an alkylene group or a heteroalkylene group. The chain structure can be either linear or branched; from the viewpoint of THRβ selectivity, the chain structure is preferably linear. The chain structure may include a partial structure, such as -O-, -C(=O)-O-, -CO-NH-, -C(=O)-, -NH-, or -S(=O)₂-, on the main chain.

From the viewpoint of THRβ selectivity, L¹ is preferably -Lⁿ- (CH₂)ₘ-. L¹¹ represents a single bond or -O-. m represents an integer of 1 to 3. From the viewpoint of THRβ selectivity, L¹¹ is preferably a single bond. From the viewpoint of THRβ selectivity, m is preferably 1 to 2, and more preferably 1.

From the viewpoint of THRβ selectivity, the compound of the present invention is preferably represented by Formula (1A): 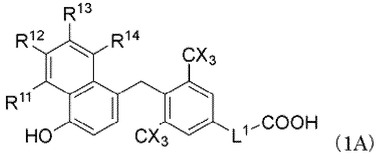 wherein
R¹¹, R¹², R¹³, and R¹⁴ are the same or different, and represent a hydrogen atom or a hydrocarbon group,
X is the same or different, and represents a halogen atom, and
L¹ represents a linker.

Examples of halogen atoms represented by X include a fluorine atom, a chlorine atom, a bromine atom, and an iodine atom. Of these, a fluorine atom is preferred.

From the viewpoint of THRβ selectivity, the compound of the present invention is more preferably represented by Formula (1AA) : 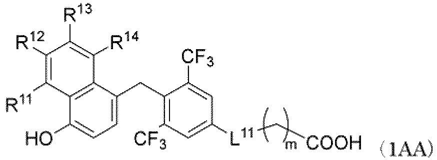 wherein
R¹¹, R¹², R¹³, and R¹⁴ are the same or different, and represent a hydrogen atom or a hydrocarbon group,
L¹¹ represents a single bond or -O-, and
m represents an integer of 1 to 3.

The salt of the compound of the present invention is not particularly limited as long as the salt is a pharmaceutically acceptable salt. The salt for use may be an acidic salt or a basic salt. Examples of acidic salts include inorganic acid salts, such as hydrochlorides, hydrobromides, sulfates, nitrates, and phosphates; and organic acid salts, such as acetates, propionates, tartrates, fumarates, maleates, malates, citrates, methanesulfonates, and p-toluenesulfonates. Examples of basic salts include alkali metal salts, such as sodium salts and potassium salts; alkaline earth metal salts, such as calcium salts and magnesium salts; salts with ammonia; and salts with organic amines, such as morpholine, piperidine, pyrrolidine, monoalkylamines, dialkylamines, trialkylamines, mono(hydroxyalkyl)amines, di(hydroxyalkyl)amines, and tri(hydroxyalkyl)amines.

The compound of the present invention can also be in the form of a hydrate or solvate. The solvent can be, for example, a pharmaceutically acceptable organic solvent (e.g., ethanol, glycerol, and acetic acid).

The compound of the present invention can be synthesized according to a method comprising, for example, the following step. 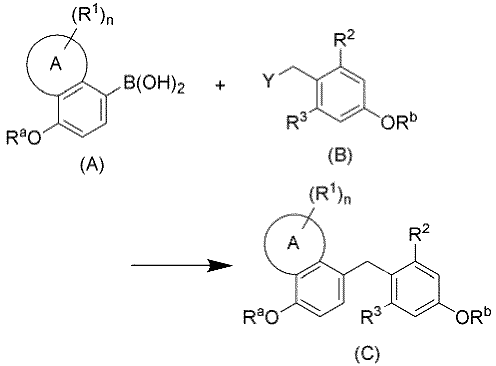 R^{a} and R^{b} are the same or different, and represent a protective group for a hydroxyl group. The protective group is not particularly limited, and examples include a methyl group, a benzyl group, a p-methoxybenzyl group, and a tert-butyl group. From the viewpoint of yield and ease of synthesis, it is preferred that R² be a methyl group, and R^{b} be a benzyl group.

Y represents a halogen atom. Examples of halogen atoms include a fluorine atom, a chlorine atom, and a bromine atom, with a chlorine atom being preferred.

Compound A and compound B for use can be commercially available products or synthesized according to a known method.

From the viewpoint of yield, ease of synthesis, or other factors, the amount of compound B used is preferably 0.2 to 1.5 mol, and more preferably 0.5 to 0.8 mol, per mole of compound A.

This reaction is typically performed in the presence of a reaction solvent. The reaction solvent is not particularly limited, and examples include toluene and benzene, with toluene being preferred. The solvent for use may be a single type or a combination of two or more.

This reaction is typically performed in the presence of a palladium catalyst, a ligand, and a base. Examples of palladium catalysts include palladium acetate and palladium chloride. Examples of ligands include triphenylphosphine. Examples of bases include potassium phosphate, potassium carbonate, and triethylamine.

In regards to temperature, the reaction can be performed with heating, at room temperature, or with cooling, and can typically be performed at 0 to 120°C. The reaction temperature is particularly preferably 70 to 100°C. The reaction time is not particularly limited and can typically be 30 minutes to 60 hours.

The progress of the reaction can be monitored according to a commonly used method, such as chromatography. After the reaction is complete, the solvent can be evaporated, and the product can be isolated and purified according to a commonly used method, such as chromatography or recrystallization. The structure of the product can be identified through elemental analysis, MS (ESI-MS) analysis, IR analysis, ¹H-NMR, ¹³C-NMR, or other methods.

In obtained compound C, -OR^{b} is deprotected and linked or replaced with -L¹-COOH or its precursor, followed by removing - OR^{b} to obtain the compound of the present invention.

### 2. Application

The active ingredient of the present invention is useful as a thyroid hormone receptor β-selective thyroid hormone analog. Thus, in one aspect, the present invention relates to a thyroid hormone analog (preferably a thyroid hormone receptor β-selective thyroid hormone analog) consisting of the active ingredient of the present invention.

Due to the usefulness describe above, the active ingredient of the present invention is also useful as a lipid metabolism improver. Thus, in one aspect, the present invention relates to a lipid metabolism improver containing the active ingredient of the present invention (which may also be referred to as "the agent of the present invention" in the present specification). The agent of the present invention can be specifically used, for example, in the improvement of conditions such as obesity, dyslipidemia (e.g., hypercholesterolemia and hyperlipidemia), and metabolic syndrome.

In the present specification, "improvement" refers to the amelioration or alleviation of symptoms or conditions, the prevention or delay of worsening of symptoms or conditions, and reversal, prevention, or delay of progression of symptoms or conditions.

The active ingredient of the present invention and the agent of the present invention can be used as pharmaceuticals, reagents, food additives, or food compositions (including health foods, health-promoting agents, and nutritional supplements).

The agent of the present invention is not particularly limited as long as the agent contains the active ingredient of the present invention, and may optionally contain other components. The other components are not particularly limited as long as they are pharmaceutically acceptable components. The other components include additives in addition to components with pharmacological action. Examples of additives include base materials, carriers, solvents, dispersants, emulsifiers, buffering agents, stabilizers, excipients, binders, disintegrants, lubricants, thickeners, humectants, colorants, fragrances, and chelating agents.

The mode of use of the active ingredient of the present invention and the mode of use of the agent of the present invention are not particularly limited. The active ingredient of the present invention and the agent of the present invention can be used, for example, in vitro (e.g., added to a culture medium for cultured cells) or in vivo (e.g., administered to or ingested by animals).

The target for applying the active ingredient of the present invention or the agent of the present invention is not particularly limited. In mammals, examples include humans, monkeys, mice, rats, dogs, cats, rabbits, pigs, horses, cows, sheep, goats, and deer. Cells for target include animal cells. There is no particular limitation on the type of cells, and examples include blood cells, hematopoietic stem cells, progenitor cells, gametes (sperm and eggs), fibroblasts, epithelial cells, vascular endothelial cells, nerve cells, liver cells, keratinocytes, muscle cells, epidermal cells, endocrine cells, ES cells, iPS cells, tissue stem cells, and cancer cells.

The agent of the present invention can be in any dosage form, such as an oral dosage form, such as tablets (including orally disintegrating tablets, chewable tablets, effervescent tablets, troches, jelly drops, etc.), pills, granules, fine granules, powders, hard capsules, soft capsules, dry syrup, liquids (including drinks, suspensions, syrup, etc.), and jellies, or a parenteral dosage form, such as injectable preparations (e.g., drip infusions (e.g., preparations for intravenous drip infusion), intravenous injections, intramuscular injections, subcutaneous injections, and intradermal injections), external preparations (e.g., ointments, cataplasms, and lotions), suppositories, inhalants, ophthalmic preparations, ophthalmic ointments, nasal drops, ear drops, and liposomal preparations.

The administration route for the agent of the present invention is not particularly limited as long as desired effects are achieved. Administration routes include oral administration, tube feeding, and enteral administration, such as enema administration; and parenteral administration, such as intravenous administration, intra-arterial administration, intramuscular administration, intracardiac administration, subcutaneous administration, intradermal administration, and intraperitoneal administration.

Examples of applications of the agent of the present invention in a food composition include a liquid, gel, or solid food, such as drinks (e.g., juice, soft drinks, tea, soup, and soy milk), salad oil, dressings, yogurt, jelly, pudding, *furikake* (Japanese dry seasoning), infant formula, cake mixes, powdered or liquid dairy products, bread, and cookies.

The content of the active ingredient in the agent of the present invention depends on factors such as the mode of use, the target of application, and the condition of the target. While not limited, the content of the active ingredient in the agent of the present invention can be, for example, 0.0001 to 100 wt%, and preferably 0.001 to 50 wt%.

The dosage of the active ingredient of the present invention or the pharmaceutical agent of the present invention administered to an animal is not particularly limited as long as it is an amount effective to exhibit drug efficacy. Typically, the dosage for oral administration on an active-ingredient weight basis is 0.1 to 1000 mg/kg body weight per day, preferably 0.5 to 500 mg/kg body weight per day, whereas the dosage for parenteral administration on an active-ingredient weight basis is 0.01 to 100 mg/kg body weight per day, and preferably 0.05 to 50 mg/kg body weight per day. The dosage above can be appropriately increased or decreased according to age, pathological conditions, symptoms, etc.

### Examples

The following provides a detailed explanation of the present invention based on Examples. However, the present invention is not limited to these Examples.

### Test Example 1: Synthesis of Compound

### 1-1: General Information

Unless otherwise specified, all materials, including anhydrous solvents, were obtained commercially and used without further purification. 2,6-Dimethyl-4-triisopropylsilyloxybenzaldehyde1, methyl 4-bromo-2,6-bis(trifluoromethyl)benzoate 2, and 4-methoxy-1-naphthaleneboronic acid 3 were prepared according to a previously reported procedure. Unless otherwise specified, all reactions were performed in an argon atmosphere using flame-dried glassware and anhydrous solvents using a standard vacuum line technique. All workup and purification procedures were performed in air using reagent-grade solvents.

Analytical thin-layer chromatography (TLC) was performed using plates precoated with E. Merck silica gel 60 F254 (0.25 mm), with visualization by UV light (254 nm) and ethanolic phosphomolybdic acid. Preparative thin-layer chromatography (PTLC) was performed using plates coated with Wakogel B5-F silica (0.75 mm), which were prepared by the present inventors. Reversed-phase HPLC was performed using a Biotage Isolera instrument equipped with a Biotage SNAP Ultra C18 Cartridge (12 g), and using MeCN/H2O as an eluent. Gas chromatography (GC) analysis was performed using a Shimadzu GC-2010 instrument equipped with an HP-5 column (30 m × 0.25 mm, Hewlett-Packard). GCMS analysis was performed using a Shimadzu GCMS-QP2010 instrument equipped with an HP-5 column (30 m × 0.25 mm, Hewlett-Packard). Column chromatography was performed using flash-grade silica (silica gel 60, spherical, particle size: 40 to 50 µm).

High-resolution mass spectra (HRMS) were obtained using a Thermo Fisher Scientific Exactive (ESI) and a JMS-T100TD instrument (DART). Nuclear magnetic resonance (NMR) spectra were recorded on a JEOL ECA600II spectrometer (¹H 600 MHz, ¹³C 150 MHz) and a JEOL ECS-400 spectrometer (¹H 400 MHz, ¹³C 100 MHz, ¹⁹F 376 MHz). ¹H NMR chemical shifts were expressed in parts per million (ppm) relative to tetramethylsilane (δ: 0.00 ppm) or the residual proton signal of CD₃OD (δ: 3.31 ppm). ¹³C NMR chemical shifts were expressed in ppm relative to CDCl₃ (δ: 77.0 ppm) or the residual proton signal of CD₃OD (δ: 49.0 ppm). Data were reported as chemical shifts, multiplicity (s = singlet, d = doublet, dd = doublet, t = triplet, dt = double triplet, q = quartet, hep = heptet, m = multiplet, br = broad signal), coupling constant (Hz), and integration.

### 1-2: Synthesis of MY-53

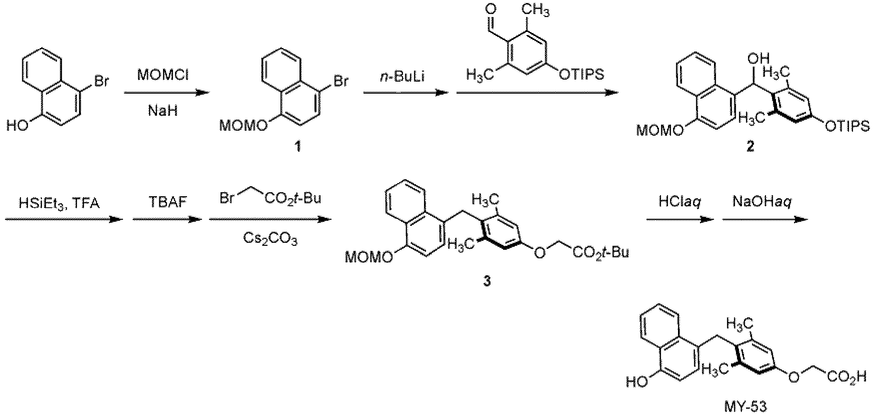

### 1-2-1: Synthesis of Compound 1

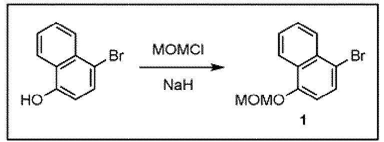

A 100 mL flask containing a magnetic stirring bar was flame-dried in a vacuum, cooled to room temperature, and then filled with argon. To the flask, 4-bromonaphthol (5.57 g, 24.9 mmol) and anhydrous DMF (20 mL) were added in a stream of argon. NaH (60% dispersion in mineral oil, 719 mg, 30.0 mmol) was added to this mixture at 0°C. After stirring at 0°C for 10 minutes, chloromethyl methyl ether (1.9 mL, 24.9 mmol) was slowly added at this temperature, and the mixture was stirred at room temperature for 16 hours. The combined extracts were dried over Na₂SO₄, and the solvent was evaporated under reduced pressure. The crude substance was purified by column chromatography (Hex/EtOAc = 20:1), thereby obtaining 1-bromo-4-(methoxymethoxy)naphthalene 1 (5.00 g, 75%) as a colorless oil.
¹H NMR (400 MHz, CDCl₃) δ 3.54 (s, 3H), 5.38 (s, 2H), 6.98 (d, J = 8.4 Hz, 1H), 7.52-7.56 (m, 1H), 7.59-7.63 (m, 1H), 7.66 (d, J = 8.0 Hz, 1H), 8.18 (d, J = 9.2 Hz, 1H), 8.29 (d, J = 8.0 Hz, 1H). ¹³C NMR (400 MHz, CDCl₃) δ 56.3, 94.7, 108.4, 114.5, 122.3, 126.1, 126.9, 127.1, 127.6, 129.5, 132.5, 152.6. HRMS (ESI) m/z calcd for C₁₂H₁₁O₂Br [M] +: 265.9937, found 265.9938.

### 1-2-2: Synthesis of Compound 2

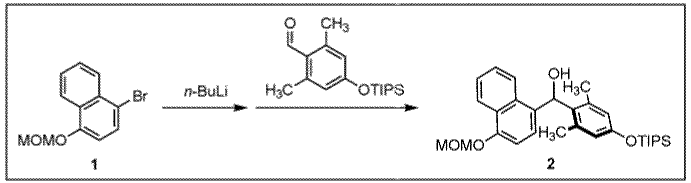

A 100 mL flask containing a magnetic stirring bar was flame-dried in a vacuum, cooled to room temperature, and then filled with argon. To the flask, 1-bromo-4-(methoxymethoxy)naphthalene (870 mg, 3.26 mmol) and anhydrous THF (10 mL) were added in a stream of argon, followed by slowly adding n-BuLi (1.6 M in hexane, 2.3 mL, 3.6 mmol) to this solution at -78°C. After stirring at 78°C for 1 hour, a solution of 2,6-dimethyl-4-triisopropylsilyloxybenzoaldehyde (1.00 g, 3.26 mmol) in THF (15 mL) was added at this temperature, and the mixture was stirred at room temperature for 16 hours. The mixture was quenched with saturated NH₄Cl(aq) and extracted with ether (3 times). The combined extracts were dried over Na₂SO₄, and the solvent was evaporated under reduced pressure. The crude substance was purified by column chromatography (Hex/EtOAc = 5:1), thereby obtaining (2,6-dimethyl-4-triisopropylsilyloxyphenyl) [4'-(methoxymethoxy)naphthyl]methanol 2 (835 mg, 52%) as a colorless oil.
¹H NMR (600 MHz, CDCl₃) δ 1.12 (d, J = 7.8 Hz, 18H), 1.26 (hep, J = 7.8 Hz, 3H), 2.24 (s, 6H), 3.52 (s, 3H), 5.35 (s, 2H), 6.60 (s, 2H), 6.74 (s, 1H), 6.92 (d, J = 7.8 Hz, 1H), 7.11 (d, J = 7.8 Hz, 1H), 7.48-7.52 (m, 2H), 8.20 (d, J = 7.8 Hz, 1H), 8.33 (d, J = 7.8 Hz, 1H). ¹³C NMR (150 MHz, CDCl₃) δ 12.7, 18.0, 21.4, 56.2, 70.5, 94.6, 106.6, 120.6, 122.5, 124.3, 125.1, 125.3, 126.4, 126.6, 130.1, 131.1, 132.6, 138.4, 152.8, 154.9. HRMS (ESI) m/z calcd for C₃₀H₄₂O₄SiNa [M+Na]+: 517.2745, found 517.2736.

### 1-2-3: Synthesis of Compound 3

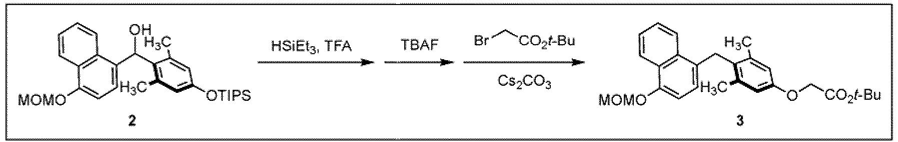

A 50 mL flask containing a magnetic stirring bar was flame-dried in a vacuum, cooled to room temperature, and then filled with argon. To the flask, (2,6-dimethyl-4-triisopropylsilyloxyphenyl)[4'-(methoxymethoxy)naphthyl]methanol (226 mg, 0.5 mmol) and anhydrous DCM (5.5 mL) were added in a stream of argon. TFA (150 L, 2 mmol) was added to this solution at 0°C, followed by adding HSiEt₃ (2.3 mL, 1.25 mmol). After stirring at 0°C for 10 minutes, the mixture was quenched with saturated Na₂CO₃(aq) and extracted with EtOAc (3 times). The combined extracts were dried over Na₂SO₄, and the solvent was evaporated under reduced pressure. The residue was dissolved in TBAF (1M in THF, 3 mL, 3 mmol), and the mixture was stirred at room temperature for 20 minutes. The mixture was quenched with water and extracted with EtOAc (3 times). The combined extracts were dried over Na₂SO₄, and the solvent was evaporated under reduced pressure. The residue was dissolved in anhydrous DMF (2 mL), and Cs₂CO₃ (489 mg, 1.5 mmol) was added. Subsequently, tert-butyl bromoacetate (60 L, 0.4 mmol) was added, and the mixture was stirred at room temperature for 12 hours. The mixture was then quenched with saturated NH₄Cl(aq) and extracted with ether (3 times). The combined extracts were dried over Na₂SO₄, and the solvent was evaporated under reduced pressure. The crude substance was purified by PTLC (Hex/EtOAc = 10:1) and GPC, thereby obtaining tert-butyl 3,5-dimethyl-4-[4'-(methoxymethoxy)naphthylmethyl]phenoxate 3 (86.4 mg, 40%) as a colorless oil.
¹H NMR (600 MHz, CDCl₃) δ 1.51 (s, 9H), 2.15 (s, 6H), 3.52 (s, 3H), 4.27 (s, 2H), 4.53 (s, 2H), 5.32 (s, 2H), 6.52 (d, J = 7.2 Hz, 1H), 6.67 (s, 2H), 6.85 (d, J = 8.4 Hz, 1H), 7.54 (t, J = 7.2 Hz, 1H), 7.61 (t, J = 7.2 Hz, 1H), 8.18 (d, J = 8.4 Hz, 1H), 8.35 (d, J = 7.2 Hz, 1H). ¹³C NMR (150 MHz, CDCl₃) δ 20.2, 28.0, 30.7, 56.2, 65.8, 82.2, 94.7, 107.6, 114.2, 122.6, 123.0, 123.4, 125.0, 126.1, 126.3, 128.3, 129.4, 133.0, 138.9, 151.5, 156.1, 168.3. HRMS (ESI) m/z calcd for C₂₇H₃₂O₅Na [M+Na]+: 459.2142, found 459.2143.

### 1-2-4: Synthesis of MY-53

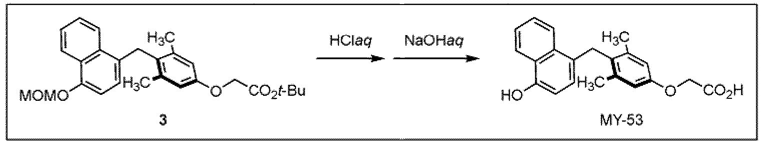

Tert-butyl 3,5-dimethyl-4-[4'-(methoxymethoxy)naphthylmethyl]-phenoxyacetate 3 (55 mg, 0.13 mmol) was added to a 50 mL flask, followed by adding i-PrOH (1.5 mL) and THF (1.5 mL). Subsequently, 2N HCl(aq) (2.0 mL) was added and stirred at 60°C for 5 hours. Brine was added, and the organic layer was extracted with EtOAc (3 × 10 mL). The extracted organic layer was washed with brine, dried over sodium sulfate, and concentrated in a vacuum. The crude substance was dissolved in MeOH (5 mL) and 2N NaOH(aq) (2.0 mL). After stirring at room temperature for 2 hours, the solvent was evaporated under reduced pressure. 1N HCl(aq) (5.0 mL) was added, and the mixture was extracted with EtOAc (3 times). The combined extracts were dried over Na₂SO₄, and the solvent was evaporated under reduced pressure. The crude substance was purified through reversed-phase HPLC (Biotage SNAP cartridge, 12 g), thereby obtaining MY-53 (23 mg, 52%) as a white solid.
¹H NMR (400 MHz, CD₃OD) δ 2.14 (s, 6H), 4.24 (s, 2H), 4.64 (s, 2H), 6.36 (d, J = 8.0 Hz, 2H), 6.56 (d, J = 8.0 Hz, 2H), 6.70 (s, 2H), 7.42-7.48 (m, 1H), 7.53-7.57 (m, 1H), 8.15 (d, J = 8.4 Hz, 1H), 8.25 (d, J = 8.4 Hz, 1H). ¹³C NMR (100 MHz, CD₃OD) δ 20.2, 31.4, 65.9, 108.3, 115.1, 123.89, 123.92, 124.5, 125.3, 126.7, 127.0, 127.2, 131.1, 134.5, 139.9, 153.0, 157.6, 173.1. HRMS (ESI) m/z calcd for C₂₁H₁₉O₄ [M-H]-: 335.1278, found 335.1284.

### 1-3: Synthesis of ZTA-245

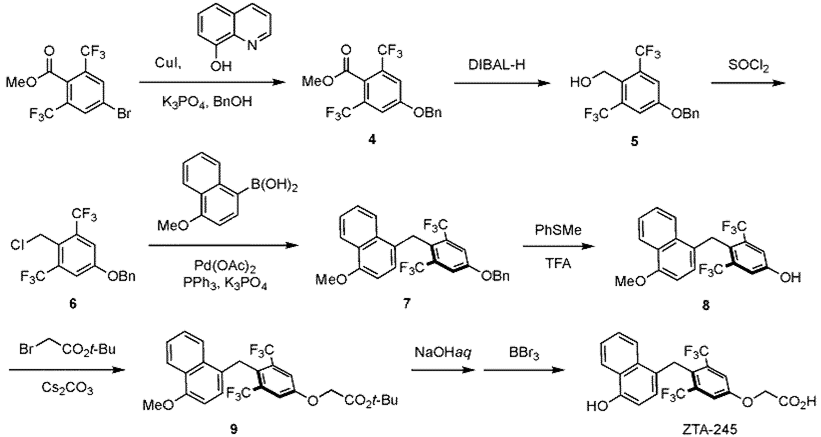

### 1-3-1: Synthesis of Compound 4

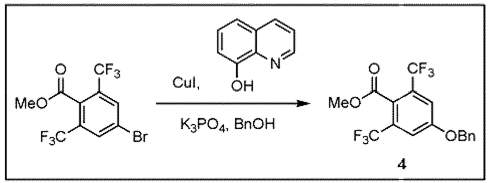

A 10 mL flask containing a magnetic stirring bar and equipped with a Teflon cap was flame-dried in a vacuum, cooled to room temperature, and then filled with argon. Methyl 4-bromo-2,6-bis(trifluoromethyl)benzoate (1.05 g, 3.0 mmol), CuI (17.1 mg, 0.09 mmol), 8-hydroxyquinoline (26.1 mg, 0.18 mmol), and K₃PO₄ (1.27 g, 6.0 mmol) were added to the flask. The flask was evacuated and backfilled with argon. Subsequently, anhydrous benzyl alcohol (3.0 mL) was added at room temperature, and the mixture was stirred at 110°C for 24 hours. Then, the mixture was quenched with saturated NH₄Cl(aq) (about 20 mL) and separated. The aqueous layer was extracted with Et₂O (2 × 15 mL), and the combined organic layers were washed with H₂O (2 × 25 mL) and brine (25 mL). The washed organic layers were then dried over sodium sulfate, filtered, and concentrated in a vacuum. The crude substance was purified by column chromatography (30:1 → 25:1 hexane:ethyl acetate), thereby obtaining methyl 4-(benzyloxy)-2,6-bis(trifluoromethyl)benzoate 4 as a white solid (0.852 g, 75%).
¹H NMR (400 MHz, CDCl₃) δ: 3.93 (s, 3H), 5.16 (s, 2H), 7.36-7.44 (m, 7H). ¹³C NMR (100 MHz, CDCl₃) δ: 53.3, 70.9, 116.0 (m), 122.6 (q, J = 272.9 Hz), 122.7, 127.6, 128.7, 128.9, 130.7 (q, J = 31.7 Hz), 134.8, 159.2, 165.6. ¹⁹F NMR (376 MHz, CDCl₃) δ: -60.0 ppm. HRMS (ESI): m/z calcd for C₁₇H₁₂F₆O₃Na([M+Na]⁺) 401.0588, found 401.0583.

### 1-3-2: Synthesis of Compound 5

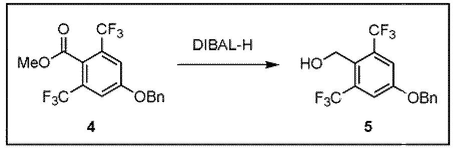

A 50 mL flask was flame-dried in a vacuum, cooled to room temperature, and then filled with argon. Methyl 4-(benzyloxy)-2,6-bis(trifluoromethyl)benzoate 4 (1.45 g, 3.82 mmol) was added to the flask, followed by adding anhydrous DCM (13 mL). The mixture was cooled to 0°C, and then diisobutylaluminum hydride (11.5 mL, 11.5 mmol, 1M solution in hexane) was added dropwise at that temperature. Thereafter, the reaction mixture was stirred at room temperature for 3 hours, after which the reaction was quenched with water (20 mL), and the mixture turned into a gel. The gel was washed with 2N HCl (3 × 50 mL), and the organic layer was extracted with EtOAc (3 × 50 mL). The extracted organic layer was washed with brine, dried over sodium sulfate, and concentrated in a vacuum. The crude substance was purified by column chromatography (Hex/EtOAc = 30:1), thereby obtaining (4-(benzyloxy)-2,6-bis(trifluoromethyl)phenyl)methanol 5 (1.45 g, 83%) as a white solid.
¹H NMR (400 MHz, CDCl₃) δ 2.03 (s, 1H), 4.83 (s, 2H), 5.11 (s, 2H), 7.33-7.43 (m, 5H), 7.47 (s, 2H). ¹³C NMR (100 MHz, CDCl₃) δ 56.6, 70.6, 116.3 (q, J = 5.7 Hz), 123.5 (q, J = 279.4 Hz), 127.6, 128.6, 128.8, 129.1, 132.9 (q, J = 31.5 Hz), 135.2, 158.0. ¹⁹F NMR (376 MHz, CDCl₃) δ: -58.3. HRMS (ESI) m/z calcd for C₁₆H₁₂O₂F₆Na [M+Na]+: 373.0634, found 373.0634.

### 1-3-3: Synthesis of Compound 6

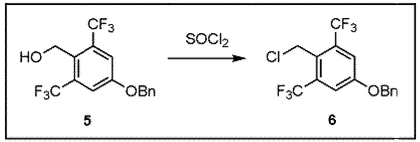

A 50 mL flask was flame-dried in a vacuum, cooled to room temperature, and then filled with argon. (4-(Benzyloxy)-2,6-bis(trifluoromethyl)phenyl)methanol 5 (1.29 g, 3.68 mmol) was added to the flask, followed by adding anhydrous DCM (3.70 mL). The mixture was cooled to 0°C, and then thionyl chloride (657 mg, 5.52 mmol) was added dropwise at that temperature. The reaction mixture was concentrated in a vacuum, and the crude substance was purified by column chromatography (Hex/EtOAc = 50:1), thereby obtaining 5-(benzyloxy)-2-(chloromethyl)-1,3-bis(trifluoromethyl)benzene 6 (1.22 g, 90%) as a clear oil.
¹H NMR (400 MHz, CDCl₃) δ 4.79 (s, 2H), 5.07 (s, 2H), 7.34-7.42 (m, 5H), 7.46 (s, 2H). ¹³C NMR (100 MHz, CDCl₃) δ 36.7, 70.7, 116.6 (q, J = 5.7 Hz), 123.3 (q, J = 279.4 Hz), 126.1, 127.6, 128.6, 128.8, 132.9 (q, J = 31.5 Hz), 135.1, 158.6. ¹⁹F NMR (376 MHz, CDCl₃) δ: -58.9. HRMS (DART) m/z calcd for C₁₆H₁₀OF₆Cl [M-H]+: 367.0324, found 367.0322.

### 1-3-4: Synthesis of compound 7

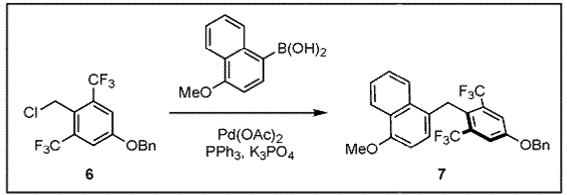

A 50 mL flask was flame-dried in a vacuum, cooled to room temperature, and then filled with argon. 5-(Benzyloxy)-2-(chloromethyl)-1,3-bis(trifluoromethyl)benzene 6 (500 mg, 1.36 mmol), (4-methoxynaphthalen-1-yl)boronic acid (411 mg, 2.03 mmol), Pd(OAc)₂ (30.4 mg, 0.14 mmol), PPh₃ (71.3 mg, 0.27 mmol), and K₃PO₄ (1.15 g, 5.42 mmol) were added to the flask. The flask was evacuated and backfilled with argon. Thereafter, anhydrous toluene (4.50 mL) was added at room temperature, and the mixture was stirred at 90°C for 16 hours. After cooling to room temperature, water (20 mL) was added, and the organic layer was extracted with ether (3 × 50 mL). The organic layer was washed with brine, dried over sodium sulfate, and concentrated in a vacuum. The crude substance was purified by column chromatography (Hex/EtOAc = 40:1), thereby obtaining 1-(4-(benzyloxy)-2,6-bis(trifluoromethyl)benzyl)-4-methoxynaphthalene 7 (516 mg, 77%) as a white solid.
¹H NMR (400 MHz, CDCl₃) δ 3.92 (s, 3H), 4.63 (s, 2H), 5.17 (s, 2H), 6.26 (d, J = 8.2 Hz, 1H), 6.57 (d, J = 8.2 Hz, 1H), 7.37-7.63 (m, 9H), 8.07 (d, J = 8.2 Hz, 1H), 8.32 (d, J = 8.2 Hz, 1H). ¹³C NMR (100 MHz, CDCl₃) δ 30.2, 55.3, 70.7, 102.9, 116.6 (q, J = 5.7 Hz), 122.5, 122.6, 123.5 (q, J = 279.4 Hz), 124.3, 124.9, 125.5, 126.6, 127.7, 128.6, 128.8, 128.9, 132.0, 133.5 (q, J = 31.4 Hz), 135.5, 153.9, 157.2. ¹⁹F NMR (376 MHz, CDCl₃) δ: -59.7. HRMS (DART) m/z calcd for C₂₇H₂₁F₆O₂ [M+H]+: 491.1446, found 491.1445.

### 1-3-5: Synthesis of Compound 8

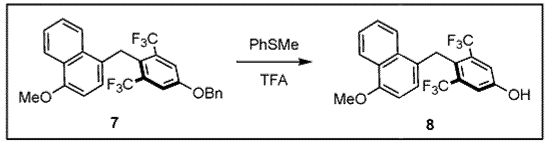

A 50 mL flask was flame-dried in a vacuum, cooled to room temperature, and then filled with argon. 1-(4-(Benzyloxy)-2,6-bis(trifluoromethyl)benzyl)-4-methoxynaphthalene 7 (127 mg, 0.26 mmol) was added to the flask, followed by adding trifluoroacetic acid (2.6 mL). Subsequently, thioanisole (1.61 g, 13.0 mmol) was added dropwise at room temperature. The reaction mixture was stirred for 22 hours, after which the reaction product was concentrated in a vacuum. The crude substance was dissolved in EtOAc (20 mL) and washed with water (3 × 50 mL). The organic layers were combined, washed with brine, dried over sodium sulfate, and concentrated in a vacuum. The crude substance was purified by column chromatography (Hex/EtOAc = 10:1), thereby obtaining 4-((4-methoxynaphthalen-1-yl)methyl)-3,5-bis(trifluoromethyl)phenol 8 as a white solid (95 mg, 92%).
¹H NMR (400 MHz, CDCl₃) δ 3.92 (s, 3H), 4.61 (s, 2H), 6.26 (d, J = 8.4 Hz, 1H), 6.57 (d, J = 8.4 Hz, 1H), 7.44 (s, 2H), 7.52 (ddd, J = 0.8, 7.6, 7.6 Hz, 1H), 7.61 (ddd, J = 0.8, 7.7, 7.7 Hz, 1H), 8.07 (d, J = 8.4 Hz, 1H), 8.32 (d, J = 8.4 Hz, 1H). ¹³C NMR (100 MHz, CDCl₃) δ 30.1, 55.3, 103.0, 117.3 (q, J = 5.7 Hz), 122.5, 122.6, 123.3 (q, J = 279.4 Hz), 124.3, 125.0, 125.5, 126.6, 128.6, 128.9, 132.0, 133.6 (q, J = 34.2 Hz), 153.9, 154.2. ¹⁹F NMR (376 MHz, CDCl₃) δ: -59.9. HRMS (ESI) m/z calcd for C₂₀H₁₃F₆O₂ [M-H]+: 399.0814, found 399.0822.

### 1-3-6: Synthesis of Compound 9

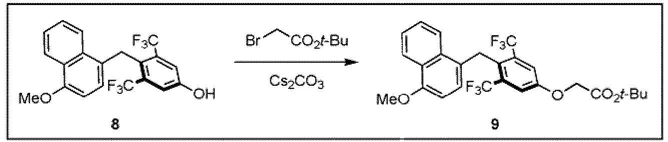

A 50 mL flask was flame-dried in a vacuum, cooled to room temperature, and then filled with argon. 4-((4-Methoxynaphthalen-1-yl)methyl)-3,5-bis(trifluoromethyl)phenol 8 (200 mg, 0.50 mmol) and cesium carbonate (705 mg, 2.0 mmol) were added to the flask, followed by adding anhydrous DMF (2.5 mL). Subsequently, tert-butyl bromoacetate (219 mg, 1.12 mmol) was added dropwise at room temperature, and the reaction product was stirred for 3 hours. Water (20 mL) was added, and the organic layer was extracted with ether (3 × 20 mL). The extracted organic layer was washed with brine, dried over sodium sulfate, and concentrated in a vacuum. The crude substance was purified by PTLC (Hex/EtOAc = 10:1), thereby obtaining tert-butyl 2-(4-((4-methoxynaphthalen-1-yl)methyl)-3,5-bis(trifluoromethyl)phenoxy)acetate 9 (196 mg, 76%) as a clear oil.
¹H NMR (400 MHz, CDCl₃) δ 1.51 (s, 9H), 3.90 (s, 2H), 4.63 (s, 3H), 6.26 (d, J = 8.4 Hz, 1H), 6.56 (d, J = 8.4 Hz, 1H), 7.49-7.53 (m, 3H), 7.60 (ddd, J = 1.2, 8.4, 8.4 Hz, 1H), 8.06 (d, J = 8.4 Hz, 1H), 8.32 (d, J = 8.4 Hz, 1H). ¹³C NMR (100 MHz, CDCl₃) δ 27.9, 30.2, 55.2, 65.8, 83.2, 102.9, 116.5 (q, J = 6.7 Hz), 122.5, 122.6, 123.4 (q, J = 279.5 Hz), 124.4, 124.9, 125.5, 126.6, 128.4, 129.7, 132.0, 133.5 (q, J = 30.5 Hz), 153.9, 156.3, 166.9. ¹⁹F NMR (376 MHz, CDCl₃) δ: -59.7. HRMS (ESI) m/z calcd for C₂₆H₂₄F₆O₄Na [M+Na]+: 537.1471, found 537.1471.

### 1-3-7: Synthesis of ZTA-245

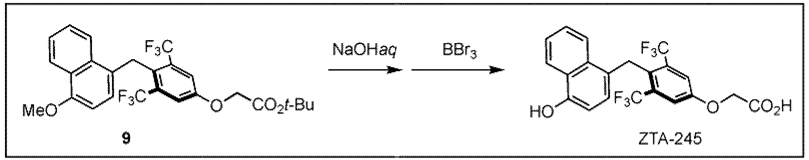

Tert-butyl 2-(4-((4-methoxynaphthalen-1-yl)methyl)-3,5-bis(trifluoromethyl)phenoxy)acetate 9 (196 mg, 0.38 mmol) was added to a 50 mL flask, followed by adding MeOH (0.6 mL) and THF (0.6 mL). Subsequently, 3N NaOH (0.6 mL) was added, and the reaction product was stirred at room temperature for 3 hours. 1N HCl (10 mL) was added, and the organic layer was extracted with EtOAc (3 × 10 mL). The extracted organic layer was washed with brine, dried over sodium sulfate, and concentrated in a vacuum. The crude substance was dissolved in anhydrous DCM (3.8 mL) and cooled to -78°C. Then, BBr₃ (1.14 mL, 1.14 mmol, 1M solution in DCM) was added dropwise, and the reaction product was stirred at -78°C for 1.5 hours. Subsequently, the reaction product was warmed to room temperature and stirred for 45 minutes. Finally, the reaction product was poured onto ice, and the organic layer was extracted with EtOAc (3 × 15 mL). The extracted organic layer was then washed with brine, dried over sodium sulfate, and concentrated in a vacuum. The crude substance was purified by reversed-phase HPLC (Biotage SNAP Cartridge, 12 g), thereby obtaining ZTA-245 (114 mg, 67%) as a white solid.
¹H NMR (400 MHz, CDCl₃) δ 4.63 (s, 2H), 4.81 (s, 2H), 6.15 (d, J = 8.8 Hz, 1H), 6.56 (d, J = 7.6 Hz, 1H), 7.51-7.56 (m, 3H), 7.60-7.64 (m, 1H), 8.07 (d, J = 8.4 Hz, 1H), 8.24 (d, J = 8.8 Hz, 1H).
¹³C NMR (100 MHz, CDCl₃) δ 30.2, 64.9, 107.8, 116.5 (q, J = 5.7 Hz), 122.3, 122.7, 123.2 (q, J = 279.4 Hz), 124.4, 125.1, 126.7, 128.8, 130.3, 132.1, 133.7 (q, J = 30.5 Hz), 149.9, 155.9, 172.5. ¹⁹F NMR (376 MHz, CDCl₃) δ: -59.7. HRMS (ESI) m/z calcd for C₂₁H₁₃F₆O₄[M-H]+: 443.0713, found 443.0720.

### 1-4: Synthesis of ZTA-261

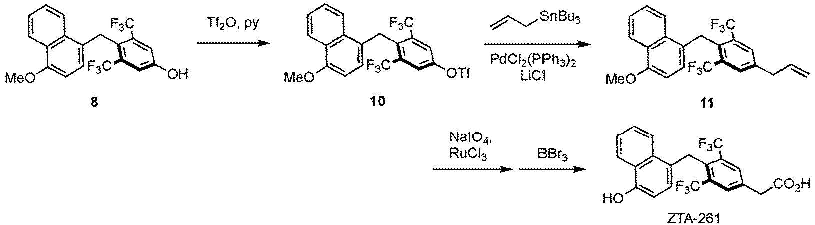

### 1-4-1: Synthesis of Compound 10

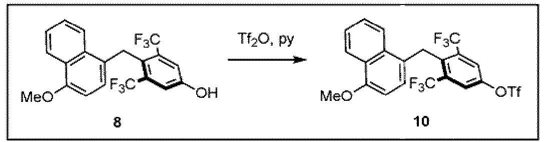

A 10 mL flask was flame-dried in a vacuum, cooled to room temperature, and then filled with argon. 4-((4-methoxynaphthalen-1-yl)methyl)-3,5-bis(trifluoromethyl)phenol 8 (123 mg, 0.31 mmol) was added to the flask, followed by adding anhydrous DCM (0.62 mL). Subsequently, pyridine (48.8 mg, 0.62 mmol) was added at room temperature, and the mixture was cooled to 0°C, followed by adding trifluoromethanesulfonic anhydride (104 mg, 0.37 mmol) dropwise. The reaction mixture was warmed to room temperature and stirred for 1 hour. The reaction product was quenched with 1N HCl (3 mL), and saturated NaHCO₃ (15 mL) was added. The organic layer was extracted with EtOAc (3 × 7 mL), washed with brine, dried over sodium sulfate, and concentrated in a vacuum. The crude substance was purified by PTLC (Hex/EtOAc = 20:1), thereby obtaining 4-((4-methoxynaphthalen-1-yl)methyl)-3,5-bis(trifluoromethyl)phenyl trifluoromethanesulfonic acid 10 (130 mg, 79%) as a white solid.
¹H NMR (400 MHz, CDCl₃) δ 3.91 (s, 3H), 4.73 (s, 2H), 6.15 (d, J = 8.4 Hz, 1H), 6.57 (d, J = 8.4 Hz, 1H), 7.54 (t, J = 8.0 Hz, 1H), 7.62 (t, J = 7.8 Hz, 1H), 7.90 (s, 2H), 8.03 (d, J = 8.4 Hz, 1H), 8.34 (d, J = 8.4 Hz, 1H). ¹³C NMR (100 MHz, CDCl₃) δ 30.7, 55.3, 102.9, 118.7 (q, J = 325.2 Hz), 122.3, 122.5 (q, J = 279.5 Hz), 122.8, 123.6 (q, J = 6.7 Hz), 124.4, 125.2, 125.6, 126.9, 127.2, 131.9, 134.8 (q, J = 32.4 Hz), 128.8, 147.4, 154.3. ¹⁹F NMR (376 MHz, CDCl₃) δ: -60.0, -72.4. HRMS (DART) m/z calcd for C₂₁H₁₄F₆O₄S [M+H]+: 533.0469, found 533.0465.

### 1-4-2: Synthesis of Compound 11

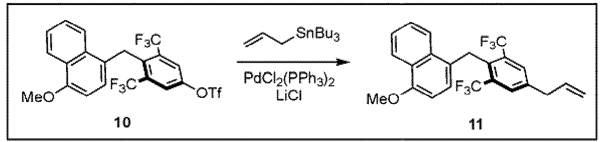

A 10 mL sealable glass container with a magnetic stirring bar contained was flame-dried in a vacuum, cooled to room temperature, and then filled with argon. 4-((4-Methoxynaphthalen-1-yl)methyl)-3,5-bis(trifluoromethyl)phenyl trifluoromethanesulfonic acid 10 (221 mg, 0.42 mmol) and LiCl (53.0 mg, 1.25 mmol) were added to the glass container. The container was evacuated and then filled with argon. Subsequently, anhydrous DMF (1.2 mL) was added at room temperature, followed by adding allyltributylstannane (137 mg, 0.42 mmol), and then adding PdCl₂(PPh₃)2 (5.8 mg, 0.008 mmol). The container was capped and sealed in a stream of argon. Water (15 mL) was added, and the organic layer was extracted with ether (3 × 15 mL). The extracted organic layer was washed with brine, dried over sodium sulfate, and concentrated in a vacuum. The crude substance was purified by PTLC (Hex/EtOAc = 20:1), thereby obtaining 1-(4-allyl-2,6-bis(trifluoromethyl)benzyl)-4-methoxynaphthalene 11 as a clear oil (120 mg, 68%).
¹H NMR (400 MHz, CDCl₃) δ 3.53 (d, J = 6.8 Hz, 1H), 3.91 (s, 3H), 5.16-5.24 (tm, 2H), 5.95-6.05 (m, 1H), 6.22 (d, J = 8.4 Hz, 1H), 6.56 (d, J = 8.0 Hz, 1H), 7.50-7.54 (m, 1H), 7.59-7.63 (m, 1H), 7.80 (s, 2H), 8.08 (d, J = 7.6 Hz, 1H), 8.32 (d, J = 8.4 Hz, 1H). ¹³C NMR (100 MHz, CDCl₃) δ 30.6, 39.4, 55.3, 102.9, 117.7, 122.5, 122.6, 123.8 (q, J = 275.9 Hz), 124.4, 125.0, 125.5, 126.7, 128.4, 130.2 (q, J = 5.8 Hz), 132.0, 132.4 (q, J = 30.7 Hz), 135.2, 135.3, 139.7, 154.0. ¹⁹F NMR (376 MHz, CDCl₃) δ: -59.4. HRMS (DART) m/z calcd for C₂₃H₁₉F₆O [M+H]+: 425.1340, found 425.1338.

### 1-4-3: Synthesis of ZTA-261

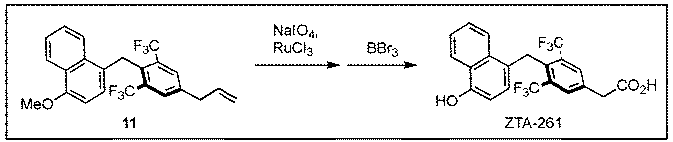

To a 10 mL flask, 1-(4-allyl-2,6-bis(trifluoromethyl)benzyl)-4-methoxynaphthalene (112 mg, 0.26 mmol) and tetrabutylammonium iodide (9.4 mg, 0.026 mmol) were added, followed by adding EtOAc (0.7 mL). Subsequently, RuCl₃-xH₂O (2.7 mg, 0.014 mmol) was added, and a solution of NaIO₄ (271 mg, 1.27 mmol) in water (2.1 mL) was added dropwise for 1 hour at room temperature. The reaction mixture was further stirred at room temperature for 4 hours. Water was added (10 mL), and the organic layer was extracted with EtOAc (3 × 10 mL). The extracted organic layer was washed with brine, dried over sodium sulfate, and concentrated in a vacuum. The crude substance was dissolved in anhydrous DCM (2.5 mL) and cooled to -78°C. Subsequently, BBr3 (0.76 mL, 0.76 mmol, 1 M solution in DCM) was added dropwise, and the reaction product was stirred at -78°C for 1.5 hours. Then, the reaction product was warmed to room temperature and stirred for 45 minutes. Finally, the reaction product was poured onto ice, and the organic layer was extracted with EtOAc (3 × 10 mL). The extracted organic layer was washed with brine, dried over sodium sulfate, and concentrated in a vacuum. The crude substance was purified by column chromatography (toluene/EtOAc/AcOH = 20:1:1) and reversed-phase HPLC (Biotage SNAP cartridge, 12 g), thereby obtaining ZTA-261 as a white solid (13.8 mg, 13%).
¹H NMR (400 MHz, CDCl₃) δ 3.85 (s, 2H), 4.70 (s, 2H), 6.13 (d, J = 8.0 Hz, 1H), 6.57 (d, J = 8.0 Hz, 1H), 7.55 (m, 1H), 7.63 (m, 1H), 7.92 (s, 2H), 8.09 (d, J = 8.4 Hz, 1H), 8.25 (d, J = 8.0 Hz, 1H). ¹³C NMR (100 MHz, CDCl₃) δ 30.8, 40.2, 107.9, 122.4, 122.8, 123.6 (q, J = 279.5 Hz), 124.47, 124.51, 125.2, 126.9, 128.7 (q, J = 5.7 Hz), 132.3, 132.9 (q, J = 31.4 Hz), 133.0, 136.8, 150.1, 175.3. ¹⁹F NMR (376 MHz, CDCl₃) δ: -59.2. HRMS (ESI) m/z calcd for C₂₀H₁₃F₆O [M-CO₂H]-: 383.0865, found 383.0876.

### Test Example 2: THRβ-selectivity Assessment (In Vitro)

The THRβ selectivity of the test compounds (T₃ (natural thyroid hormone), GC-1 (known THRβ-selective agonist), and ZTA-261) was assessed using a radioligand-displacement assay. Specifically, the assessment was performed as described below by using full-length human THRα and THRβ synthesized by in vitro transcription-translation.

2 µL of in vitro translation mixture of either THRα or THRβ was mixed with 0.5 nM [¹²⁵I]-T₃ (7.4 MBq/mL, 81.4 TBq/mmol, PerkinElmer) and a non-radioactive test compound serially diluted from 10⁻⁵ to 10⁻¹¹ in 100 µL of E400 buffer (20 mM potassium phosphate buffer, pH of 8.0, 400 mM NaCl, 0.5 mM EDTA, 1 mM MgCl₂, 1 mM monothioglycerol, 500 µg/mL calf thymus histone). The reaction mixture was incubated overnight at 4°C. A nitrocellulose membrane (Optitran, 0.45 µm in pore size, Whatman) pre-blocked with E-400 buffer was placed in a dot-blot apparatus (Minifold I, GE Healthcare), and the reaction mixture was filtered through the membrane, followed by washing three times with 200 µL of E-400 buffer. The membrane was removed from the manifold, air-dried for 5 minutes, wrapped in plastic film, and exposed to an imaging plate (BAS IP MS 2040 E, FUJI FILM) for 1 hour. Radioactive signals were scanned using a Typhoon FLA9000 (GE Healthcare) and quantified by using Image Quant TL software (GE Healthcare). The IC₅₀ value of each competitor drug was calculated by fitting the dose response data to a one-site competition model using GraphPad Prism version 7.0 (GraphPad Software).

Fig. 1 and Table 1 show the results. T₃, GC-1, and ZTA-261 were shown to bind with similar affinity to THRβ, with IC₅₀ values of 2.9 nM, 3.1 nM, and 5.8 nM, respectively (Fig. 1 and Table 1). The key difference between these compounds is seen in their binding to THRα. While T₃ binds to THRα with almost the same affinity as it does to THRβ (IC₅₀ of 4.2 nM), GC-1 and ZTA-261 show much lower affinity for THRα (IC₅₀ of 93 and 410 nM, respectively). The affinity of GC-1 and the affinity of ZTA-261 for THRα are about 30 times and about 70 times lower than their affinity for THRβ, respectively (Fig. 1 and Table 1). The results indicate that ZTA-261 is a novel analog showing higher THRβ selectivity than a known THRβ-selective agonist (GC-1).

**Table 1**

| **Competitor** | **hTHR *α*** | | **hTHR *β*** | |
|---|---|---|---|---|
| | **IC₅₀ (nM)** | **95% CI (nM)** | **IC₅₀ (nM)** | **95% CI (nM)** |
| **T₃** | 4.2 | 2.3 to 7.5 | 2.9 | 2.4 to 3.5 |
| **GC-1** | 93 | 66 to 1.3x10² | 3.1 | 2.5 to 3.8 |
| **ZTA-261** | 4.1x10² | 1.9x10² to 8.0x10² | 5.8 | 4.6 to 7.3 |

### Test Example 3: THRβ-selectivity Assessment (In Vivo)

The THRβ selectivity of the test compounds (T₃ (natural thyroid hormone), GC-1 (known THRβ-selective agonist), GC-24 (known THRβ-selective agonist), ZTA-261, ZTA-245, and MY-53) was assessed using a luciferase assay. Specifically, the procedure performed is described below.

The cells used and cell culture were as follows. GloResponse 9XGAL4UAS-luc2P HEK293 cells (Promega) were stably transfected with pFN26A (BIND) (Promega) containing the coding region of the ligand-binding domain of THRα or THRβ. The cells were cultured in DMEM supplemented with 10% FBS, 200 µg/mL Hygromycin, and 750 µg/mL G418 in 90 mm cell culture dishes at 37°C with 5% CO₂. The cells were subcultured every 3 days and used in a luciferase reporter assay.

The cells were suspended in an assay medium (DMEM supplemented with 5% charcoal/dextran-treated FBS, 25 mM HEPES, and a 4 mM L-Alanyl-L-glutamine solution) and seeded at 2 × 10⁴ cells/well in 96-well plates coated with poly-L-lysine. After incubation at 37°C with 5% CO₂ for 24 hours, each test compound was added at various concentrations. The following day, firefly luciferase luminescence was measured using a Dual-Glo Luciferase Assay System (Promega) with a microplate reader (SpectraMax i3, Molecular Devices). The luminescence of Renilla luciferase was used as an internal standard. The EC₅₀ value of each test compound was calculated by fitting the data to a Sigmoidal dose-response model using GraphPad Prism version 7.0 (GraphPad Software).

The results indicate that as seen in Test Example 2, ZTA-261 showed higher THRβ selectivity than a known THRβ-selective agonist (GC-1). ZTA-245 and MY-53 were also found to show THRβ selectivity in a similar manner.

### Test Example 4: Assessment of Effect of Test Compounds on Liver Lipids

Lipid droplets are organelles for storing neutral lipids, such as triglycerides and cholesterol esters. Excessive accumulation of lipid droplets in hepatocytes is characteristic of non-alcoholic fatty liver disease (NAFLD). To evaluate the effect of the test compounds (T₃, GC-1, and ZTA-261) on liver lipids, liver sections were stained with Oil Red O to visualize the accumulation of lipid droplets in hepatocytes. Specifically, the procedure performed is described below.

Male 7-week-old C57BL/6J mice were individually housed in cages (Innocages, ORIENTAL GIKEN) and kept warm at 23°C with a 12-hour light-dark cycle starting at 8:00 AM. After 1 week of acclimation, the animals were given either a normal diet (ND; 10 kcal% fat, D12450B, Research Diets) or a high-fat diet (HFD; 60 kcal% fat, D12492, Research Diets). After 8 weeks of being kept on ND or HFD, the mice were intraperitoneally injected with saline, T₃ (1 µmol/kg-day), GC-1 (1 µmol/kg-day), or ZTA-261 (1 µmol/kg-day) for 3 weeks. The injection was given 6 hours after lights on. Food consumption and body weight were measured weekly. Food and water were provided to the animals ad libitum. After 3 weeks of daily injections, whole blood was collected under isoflurane anesthesia, and the heart and liver were harvested. The liver was frozen and stored at -80°C.

20 µm sections were prepared from the frozen liver tissues using a cryostat (CM3050 S, Leica). The sections were mounted on silane-coated glass slides, dried at room temperature, and stored at -80°C until use. Before staining, the sections were fixed with 1% PFA/1 × PBS, pH of 7.4 (Gibco), for 5 minutes, then immersed in 1 × PBS for 5 minutes, distilled water for 5 minutes, and a 60% 2-propanol solution for 1 minute. The Oil Red O staining solution was prepared by mixing 0.36 g of Oil Red O (Sigma) with 120 mL of 2-propanol and 80 mL of distilled water, letting it stand at room temperature for 1 hour, filtering the solution, and then leaving it overnight at 37°C. The sections were immersed in the Oil Red O staining solution at 37°C for 15 minutes, and then rinsed in 60% 2-propanol for 1 minute and in distilled water for 1 minute. Thereafter, the sections were counterstained with Meyer's hematoxylin. The sections were observed under a microscope (BX43; Olympus) at 100x magnification, and digital images were captured using cellSens software (Olympus). The area of red-stained lipid droplets was quantified using Image J (NIH), and the percentage of the stained area in the total image area was calculated. Three sections per mouse were analyzed.

Fig. 2 shows the results. Treatment with T₃, GC-1, or ZTA-261 significantly reduced the Oil Red O-positive area compared to the vehicle. Of these test compounds, ZTA-261 was the most effective, reducing the Oil Red O-positive area to a level comparable to the ND-administered group.

### Test Example 5. Assessment of Impact on Gene Expression

To investigate the mechanism by which ZTA-261 promotes lipid metabolism, the expression of genes involved in lipid metabolism in the liver was studied by using reverse transcription quantitative PCR (RT-qPCR) analysis upon administration of the compounds at 1 µmol/kg BW/day. Specifically, the procedure performed is described below.

Total RNA was extracted from the frozen liver tissues (Test Example 4) using a QIAzol reagent (Qiagen) according to the manufacturer's protocol. The extracted RNA was stored at -80°C until use. cDNA was synthesized by performing qPCR reaction by using QuantStudio 3 (Applied Biosystems) in 20 µL of a reaction mixture containing 2 µL of cDNA, 10 µL of TB Green Premix Ex Taq II (TAKARA), 0.4 µL of ROX Dye II (TAKARA), and 0.4 µM of gene-specific primers (Pnpla2 gene). GAPDH was used as an internal control. Relative expression levels were calculated according to a known method.

Fig. 3 shows the results. Pnpla2, which encodes adipose triglyceride lipase (ATGL), which catalyzes the hydrolysis of triglycerides, increased significantly in the group treated with GC-1 and in the group treated with ZTA-261. In particular, the degree of increase with ZTA-261 was more pronounced than with GC-1.

Additionally, the results of further testing indicate that the administration of T₃, GC-1, or ZTA-261 promoted the cellular uptake of LDL, increased the expression of Ldlr, which encodes the low-density lipoprotein receptor that promotes cholesterol degradation, and decreased the expression of Srebplc, which encodes a transcription factor that activates the synthesis of fatty acids and triglycerides. These results suggest that GC-1 and ZTA-261 promote lipid metabolism by activating similar metabolic pathways.

### Test Example 6: Safety Assessment 1

To evaluate the hepatotoxicity of ZTA-261 in vivo, serum levels of alanine aminotransferase (ALT), a marker of hepatocellular necrosis, were measured. Specifically, the procedure performed is described below.

The blood samples collected in Test Example 4 were left to stand at room temperature for 1 hour, and then centrifuged at 4°C, 800 × g, for 15 minutes, followed by collecting the supernatant. The serum ALT levels were measured using an Alanine Aminotransferase (ALT or SGPT) Activity Colorimetric/Fluorometric Assay Kit (BioVision) according to the manufacturer's protocol.

Fig. 4A shows the results. When GC-1 was administered at 1 µmol/kg BW/day, a significant increase in serum ALT levels was observed. However, mice treated with ZTA-261 did not show a significant increase in serum ALT levels compared to the vehicle at either a low dose (0.1 µmol/kg BW/day) or a high dose (1 µmol/kg BW/day), indicating that ZTA-261 is less hepatotoxic than GC-1.

### Test Example 7: Safety Assessment 2

Left ventricular hypertrophy is often associated with hyperthyroidism caused by increased heart rate mediated by hTHRα. Therefore, hTHRβ-selective analogs are expected to avoid this adverse effect on the heart. To examine whether cardiac hypertrophy was induced, the weight of the heart collected in Test Example 4 was measured.

Fig. 4B shows the results. Administration of T₃ and administration of a high dose of GC-1 (1 µmol/kg BW/day) significantly increased heart weight. No such effect was observed in the hearts of animals treated with either a high dose (1 µmol/kg BW/day) or low dose (0.1 µmol/kg BW/day) of ZTA-261.

### Test Example 8: Safety Assessment 3

To test whether the increase in heart weight due to treatment with T₃ or GC-1 was caused by an increase in the size of cardiomyocytes in the left ventricle, heart sections were stained with hematoxylin-eosin, and the transnuclear width of left ventricular cardiomyocytes was measured. Specifically, the procedure performed is described below.

The cardiac tissues collected in Test Example 4 were fixed, dehydrated, and paraffin-infiltrated using an automatic fixation and encapsulation device (Sakura Finetek Japan). Paraffin blocks were prepared using a Tissue Tech TEC 6 (Sakura Finetek Japan), and 5 µm thick transverse sections were prepared using a microtome (Leica). The sections were deparaffinized, rehydrated, and then immersed in a Meyer's hematoxylin solution (FUJIFILM Wako Pure Chemical Industries, Ltd.) and in an eosin solution. Thereafter, the sections were dehydrated and mounted using Entellan new (Merck). The sections were observed under a microscope (BX43; Olympus) at 400x magnification, and digital images were captured using cellSens (Olympus). One hundred cells were randomly selected from the left ventricle of each mouse, and their transnuclear width was measured using Image J (NIH) according to a previously reported method.

Fig. 4C shows the results. Administration of T₃ and administration of a high dose (1 µmol/kg BW/day) of GC-1 increased the width of cardiomyocytes, but no significant increase was observed in animals treated with ZTA-261. These results indicate that the cardiac toxicity of ZTA-261 was lower than that of not only T₃ but also THRβ-selective GC-1.

### Test Example 9: Assessment of Effect on Bone Metabolism

The effect of the test compounds on bone metabolism was investigated. Specifically, bone structure parameters (bone volume: BV/TV, trabecular number: Tb.N, trabecular separation: Tb.Sp, trabecular thickness: Tb.Th, connectivity density: Conn.D) were measured using thin sections prepared from the bones of mice in Test Example 4.

Fig. 5 shows the results. Administration of T₃ and administration of GC-1 significantly worsened the bone structure parameters, but no significant changes were observed with administration of ZTA-261.

## Claims

1. A compound represented by Formula (1), a salt thereof, or a solvate thereof: 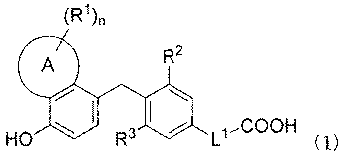 wherein
A represents an aromatic ring,
R¹ is the same or different, and represents a hydrocarbon group,
n represents an integer of 0 to 6,
R² and R³ are the same or different, and represent a trihalomethyl group or an alkyl group, and
L¹ represents a linker.

2. The compound, salt thereof, or solvate thereof according to claim 1, wherein R² and R³ are the same or different, and represent a trihalomethyl group or a C₁-C₆ alkyl group.

3. The compound, salt thereof, or solvate thereof according to claim 1, wherein the compound is represented by Formula (1A): 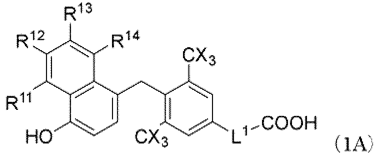 wherein
R¹¹, R¹² , R¹³, and R¹⁴ are the same or different, and represent a hydrogen atom or a hydrocarbon group,
X is the same or different, and represents a halogen atom, and
L¹ represents a linker.

4. The compound, salt thereof, or solvate thereof according to claim 3, wherein the number of atoms that constitute the main chain of the linker is 1 to 4.

5. The compound, salt thereof, or solvate thereof according to claim 3, wherein the compound is represented by Formula (1AA): 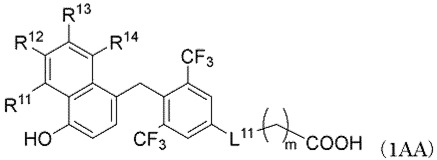 wherein
R¹¹, R¹², R¹³, and R¹⁴ are the same or different, and represent a hydrogen atom or a hydrocarbon group,
L¹¹ represents a single bond or -O-, and
m represents an integer of 1 to 3.

6. A thyroid hormone analog consisting of the compound, salt thereof, or solvate thereof of any one of claims 1 to 5.

7. A lipid metabolism improver comprising the compound, salt thereof, or solvate thereof of any one of claims 1 to 5.

8. The lipid metabolism improver according to claim 8, for use in the improvement of at least one condition selected from the group consisting of obesity, dyslipidemia, and metabolic syndrome.
